# EUROPEAN PATENT SPECIFICATION

(11) **EP 3 145 460 B1**
(45) Date of publication and mention of the grant of the patent: **23.06.2021**
(21) Application number: 15722888.3
(22) Date of filing: 06.05.2015
(51) Int. Cl.: A61F 11/08

(54) **PUSH-TO-FIT EARPLUG HAVING GEOMETRIC FLANGE FEATURES**
SCHIEBESITZ-OHRSTÖPSEL MIT GEOMETRISCHEN FLANSCHMERKMALEN
BOUCHON D'OREILLE DOTÉ D'ÉLÉMENTS BRIDES GÉOMÉTRIQUES À AJUSTER EN POUSSANT

(30) Priority: 20.05.2014 US 201414282252
(43) Date of publication of application: 29.03.2017
(73) Proprietor: 3M Innovative Properties Company, St. Paul, MN 55133-3427 (US)
(72) Inventor: HAMER, Jeffrey L., Saint Paul, Minnesota 55133-3427 (US); TEETERS, Kenneth F., Saint Paul, Minnesota 55133-3427 (US); THOMAS, Ravi, Saint Paul, Minnesota 55133-3427 (US)
(74) Representative: Mathys & Squire
(86) International application number: PCT/US2015/029359
(87) International publication number: WO 2015/179128

(56) References cited:
- US-A- 1 406 425
- US-A- 3 896 801
- US-A1- 2005 274 568
- US-A1- 2008 314 393
- None

## Description

### Technical Field

This disclosure relates to a hearing protection device, in particular a push-to-fit earplug having a flange including an interior surface having a plurality of inwardly projecting geometric features that affect compression and flexing of the flange.

### Background

The use of hearing protective and noise attenuating devices are well known, and various types of devices have been considered. Such devices include earplugs and semi-aural devices partially or completely constructed of foam or rubber materials that are inserted into, or placed over, the ear canal of a user to physically obstruct the passage of sound waves into the inner ear.

Compressible or "roll-down" type earplugs generally comprise a compressible, resilient body portion and may be made of suitable slow recovery foam materials. The earplug may be inserted into the ear canal of a user by first rolling it between fingers to compress the body portion, then pushing the body portion into the ear canal, and subsequently allowing the body portion to expand to fill the ear canal.

Push-to-fit type earplugs have also been considered, and may include a compressible attenuating portion and a stiff portion that extends from the attenuating portion. To insert a push-to-fit type earplug, the user grasps the stiff portion and pushes the attenuating portion into the ear canal with an appropriate level of force. The attenuating portion compresses as it is accommodated in the ear canal. Push-to-fit earplugs may allow the earplug to be quickly and easily inserted in an ear canal, and may promote hygiene by minimizing contact with the attenuating portion of the earplug prior to insertion US2008314393 describes a self-conforming sound attenuation earplug, including: a stem, at least one support joined with the stem and extending radially outward from the stem, and a shell engaging the support and encircling the stem to form a space between the shell and the stem. At least one of the support material and the shell material is a deformable-resilient material.

### Summary

The present disclosure relates to an earplug, such as a push-to-fit earplug. The invention is defined by the claims. In an exemplary embodiment, an earplug includes a stem and a sound attenuating body attached to the stem. The sound attenuating body includes a leading end, a base end, a longitudinal axis extending between the leading end and the base end, and a flange extending at least partially over the stem and including an exterior flange surface and an interior flange surface having a plurality of one or both of protrusions or recesses. The earplug further includes a flange cavity including a continuous volume around a perimeter of the stem between the interior flange surface and the stem. A distance between the interior and exterior flange surfaces varies around a perimeter of the flange at a plane intersecting the flange transverse to the longitudinal axis. In some exemplary embodiments, the flange has a minimum flange thickness (Fmin) and a maximum flange thickness (Fmax) between the exterior flange surface and the interior flange surface at the plane intersecting the flange transverse to the longitudinal axis, and 1.5 mm < (Fmax) < 5 mm and 0.5 mm < (Fmin) < 1.5 mm.

In another exemplary embodiment, the present description provides a hearing protection device including a stem comprising a core made of a first material and an outer layer made of a second material, and a sound attenuating body attached to the stem. The sound attenuating body includes a leading end, a base end, a longitudinal axis extending between the leading end and the base end, and a flange extending at least partially over the stem including an exterior flange surface and an interior flange surface having a plurality of one or both of protrusions or recesses. The hearing protection device further includes a flange cavity including a continuous volume around a perimeter of the stem between the interior flange surface and the stem. The flange has a minimum flange thickness (Fmin) and a maximum flange thickness (Fmax) between the exterior flange surface and the interior flange surface at a plane passing through the flange transverse to the longitudinal axis, and the flange comprises protrusions that exhibit a width (w) measured between two adjacent locations of minimum flange thickness (Fmin) at the plane intersecting the flange transverse to a longitudinal axis of the stem. In an exemplary embodiment, 1.5 mm < (Fmax) < 5 mm, 0.5 mm < (Fmin) < 1.5 mm, and 2 mm < (w) < 8 mm.

The above summary is not intended to describe each disclosed embodiment or every implementation. The Figures and the Detailed Description, which follow, more particularly exemplify illustrative embodiments.

### Brief Description of Drawings

The disclosure may be further explained with reference to the appended Figures, wherein like structure is referred to by like numerals throughout the several views, and wherein:
FIG. 1 is a front perspective view of an exemplary earplug according to the present description.
FIG. 2 is a cross-sectional view of an exemplary earplug according to the present description.
FIG. 3 is a rear view of an exemplary earplug according to the present description including a plurality of splines.
FIGS. 4a - 4d are rear plan views of exemplary earplugs according to the present description showing flanges having various spline shapes.
FIG. 5 is a side view of an exemplary earplug according to the present description showing an exemplary sound attenuating body profile.
FIG. 6a is a perspective view of an exemplary earplug according to the present description having a sound attenuating body including a tip cavity and an array of cavities in a tip region.
FIG. 6b is a cross-sectional view of an exemplary earplug according to the present description including a tip cavity and an array of cavities in a tip region.

While the above-identified figures set forth various embodiments of the disclosed subject matter, other embodiments are also contemplated. In all cases, this disclosure presents the disclosed subject matter by way of representation and not limitation. It should be understood that numerous other modifications and embodiments can be devised by those skilled in the art which fall within the scope and spirit of the principles of this disclosure.

### Detailed Description

An earplug that provides hearing protection for a user is provided herein. An earplug according to the present disclosure includes a stem and a sound attenuating body having a flange. When inserted into the ear canal of a user, the flange is able to at least partially collapse into a continuous volume defined between an interior surface of the flange and the stem. The interior surface of the flange includes a plurality of geometric features, such as protrusions and/or recess. The geometric features affect collapse and/or compression of the flange such that undesirable creasing or buckling of the flange is minimized. An earplug having geometric flange features as described herein facilitates an earplug that is comfortable to wear and minimizes undue noise leakage into an ear canal by limiting creasing or buckling of the flange.

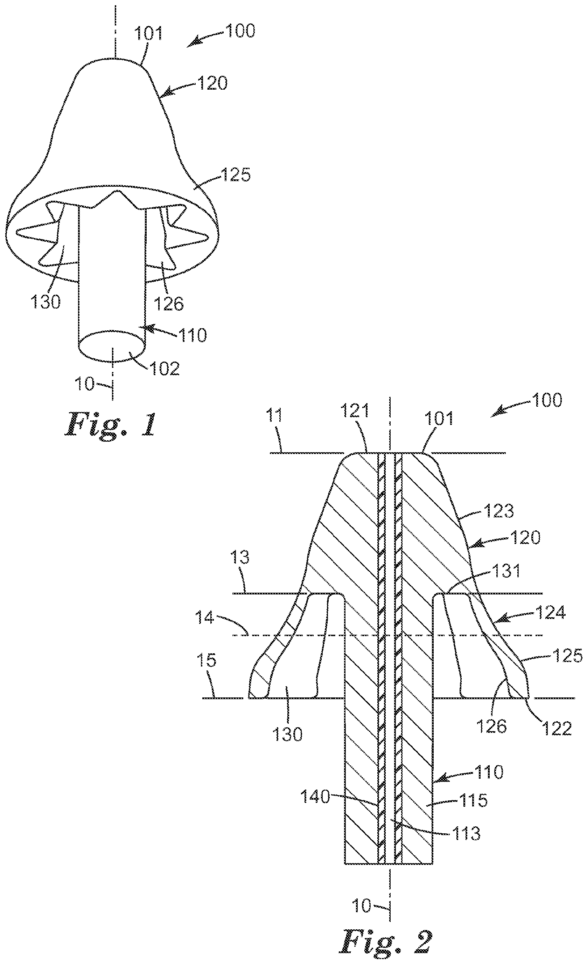

FIGS. 1 through 3 show an exemplary push-to-fit earplug 100 including a stem 110 and sound attenuating body 120 and having first and second ends 101 and 102. Sound attenuating body 120 is configured for at least partial insertion into the ear canal of a user to attenuate the passage of sound into the ear canal. During insertion of earplug 100, stem 110 may serve as a handle which may be gripped by a user. Earplug 100, and specifically sound attenuating body 120, is brought proximate to the user's ear and inserted into the ear canal. Sound attenuating body 120 compresses and/or collapses as it is positioned, and stem 110 provides sufficient stiffness to facilitate insertion. In use, sound attenuating body 120 is positioned substantially within an ear canal to block the passage of sound and stem 110 extends outwardly from the ear canal to provide a handle to remove the earplug.

In an exemplary embodiment, sound attenuating body 120 includes a leading end 121, a base end 122, a tip region 123 and a flange 124 extending at least partially over stem 110. Tip region 123 is located rearwardly of leading end 121 and flange 124 is located between tip region 123 and base end 122. Sound attenuating body 120 defines a flange cavity 130 between stem 110 and sound attenuating body 120. In an exemplary embodiment, flange cavity 130 is defined at least in part by stem 110, sound attenuating body 120, and a flange cavity bottom 131. Flange cavity bottom 131 may be formed by a portion of tip region 123, where sound attenuating stem 110 and sound attenuating body 120 intersect, for example.

Certain features of an earplug according to the present description may be understood in view of various reference planes defined relative to earplug 100 and shown in Figure 2. A longitudinal axis 10 extends between leading end 121 and base end 122 of sound attenuating body 120. A leading end plane 11 passes across an outermost tip of leading end 121, and/or first end 101, of earplug 100 transverse to the longitudinal axis. A cavity plane 13 intersects earplug 100 at a forward-most portion of flange cavity 130, and a flange end plane 15 intersects earplug 100 transverse to longitudinal axis 10 at base end 122 of flange 124.

In an exemplary embodiment, flange cavity 130 may be described as opening towards second end 102 of earplug 100 or, for example, away from tip region 123 of sound attenuating body 120. Flange 124 may be defined as that portion of sound attenuating body 120 that is located below the cavity plane 13.

In an exemplary embodiment, flange 124 is not attached to stem 110 within flange cavity 130, or between cavity plane 13 and base end 122, and only attaches to stem at and/or above cavity plane 13. Because flange 124 is connected to a remainder of sound attenuating body 120 and/or stem 110 only near one end, flange cavity 130 includes a continuous volume around stem 110. That is, in an exemplary embodiment, an interior flange surface 126 of flange 124 does not contact stem 110 when in a neutral, uncompressed configuration such as when not positioned in an ear canal. In an exemplary embodiment, the flange cavity includes a continuous uninterrupted volume around a perimeter of at least a portion stem 110. Flange 124 may deflect inwardly as earplug 100 is advanced into an ear canal and/or is positioned therein, and in some embodiments interior flange surface 126 may at least partially contact stem 110. In various exemplary embodiments, deflection and/or compression of flange 124 may improve insertion, comfort, and sound attenuation, and may be controlled by the materials, geometry, and configuration of earplug 100, as described further herein.

Interior flange surface 126 of flange 122 includes a plurality of geometric features. The geometric features may be configured to promote buckling and/or folding of interior flange surface 126, and/or stretching and tensioning of exterior flange surface 125, when sound attenuating body 120 is compressed during insertion into an ear canal. For example, geometric features of interior surface 126 may include a plurality of one or both of protrusions or recesses such that flange 124 exhibits different thicknesses at various locations around a perimeter of flange 124. In an exemplary embodiment, a thickness of flange 124 between exterior and interior flange surfaces 125, 126 varies around flange 124 at a flange plane, such as a plane 14 for example, oriented transverse to longitudinal axis 10 and passing through flange 124 and flange cavity 130 (i.e. between cavity plane 13 and base end plane 15). Accordingly, the geometric features are configured to result in locations of stress concentration where buckling and/or folding may be most likely to occur to control compression and/or collapse of flange 124.

In an exemplary embodiment, flange 124 includes a plurality of geometric features in the form of splines 150 spaced about flange 124 and extending from base end 122 of flange 124 at least partially towards bottom 131 of flange cavity 130. Splines 150 provide locations of varying thickness of flange 124 spaced about interior flange surface 125. Relatively thinner locations are selectively positioned to result in stress concentrations where flange 124 may fold, flex, and/or buckle at interior flange surface 126 when sound attenuating body 120 is compressed. In an exemplary embodiment, splines 150 are symmetrically or uniformly spaced about interior flange surface 125 such that flange 124 may react to compressive forces in a substantially uniform manner.

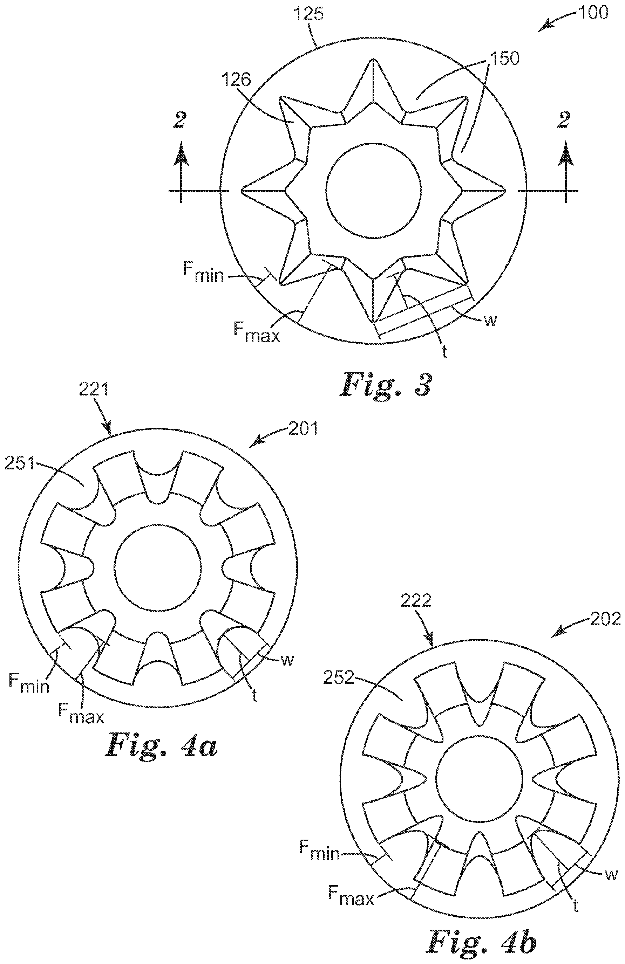

In various exemplary embodiments, such as shown in FIG. 3, flange 124 may be characterized by a minimum flange thickness (Fmin) and a maximum flange thickness (Fmax) between exterior flange surface 125 and interior flange surface 126. For example, at a plane intersecting flange 124 transverse to longitudinal axis 10, flange 124 has a minimum flange thickness (Fmin) at valleys 152 between splines 150, and a maximum flange thickness (Fmax) at a location where splines extend most inwardly towards stem 110. Flange thickness may be measured at a plane passing through flange 124 transverse to longitudinal axis 10, such as plane 14, and may vary between bottom 131 of flange cavity 130 and base end 122 of flange 124. In an exemplary embodiment, splines 150 exhibit a minimum flange thickness (Fmin) between 0.20 mm and 3 mm, 0.5 mm and 1.5 mm, 1.0 mm and 1.3 mm, or about 1.2 mm and a maximum flange thickness (Fmax) between 1.2 and 6 mm, 1.5 and 5, 2.0 and 4.5, or about 4.0 mm.

Splines 150 may be characterized by a spline width (w) and a spline thickness (t). Spline width (w) is a minimum distance between two adjacent locations of a minimum flange thickness (Fmin). For example, base width (w) of earplug 100 is a distance between adjacent valleys 152 of splines 150. In an exemplary embodiment, spline thickness (t) is a marginal flange thickness resulting from the presence of a protrusion or recess, for example spline 150, and is equal to the difference of maximum flange thickness (Fmax) less minimum flange thickness (Fmin). In an exemplary embodiment, splines 150 exhibit a width (w) at a location at least one plane passing through flange 124 transverse to longitudinal axis 10 of between 2 mm and 8 mm, 3 mm and 7 mm or of about 6 mm, and a thickness (t) between 0.5 mm and 4 mm, 1 mm and 3 mm, or of about 2.75 mm. An earplug 100 having a flange 124 exhibiting such dimensions may provide a desired level of comfort by reducing a force exerted when positioned in an ear canal of a user and/or promote desired attenuation by minimizing folds or creases in exterior flange surface 125.

Earplug 100 may include any suitable number of protrusions, recesses, splines, or other geometric features. In various exemplary embodiments, earplug 150 includes between 4 and 24, 6 and 18, or about 8 splines 150. In an exemplary embodiment, an earplug 100 include 8 splines having a generally triangular cross-section may be configured to have a minimum flange thickness (Fmin) of about 1.2 mm, a maximum flange thickness (Fmax) of about 4.0 mm, a width (w) of about 6 mm, and a thickness (t) of about 2.75 mm.

Geometric features such as splines 150 have a width aspect ratio (Wr) of (w/Fmax) at a location of at least one plane passing through flange 124 transverse to longitudinal axis 10. In various exemplary embodiments, splines 150 are characterized by a width ratio (Wr) between about 0.5 and 3, 0.75 and 1.75, or of about 1.5.

Geometric features such as splines 150 further have a ratio of spline width (w) to spline thickness (t) that may be characterized as an aspect ratio (R). In an exemplary embodiment aspect ratio (R) of (w/t) is between 0.75 and 4, 1 and 3, or about 1.5. An earplug 100 having an aspect ratio (R) within such ranges provides a combination of comfort and sound attenuation when positioned for use in an ear canal of a user.

Maximum flange thickness (Fmax), minimum flange thickness (Fmin), spline width (w) and spline thickness (t) may be uniform or vary between flange cavity bottom 131 and base end 122 of flange 124. In an exemplary embodiment, spline base width (w) and spline thickness (t) are largest at a location near base end 122 of flange 124 and decrease at locations closer to flange cavity bottom 131. In an exemplary embodiment, spline width (w) and spline thickness (t) uniformly decrease from base end 122 towards bottom 131 such that aspect ratio (R) remains substantially constant. In another exemplary embodiment, spline width (w) and spline thickness (t) are constant for a distance before uniformly decreasing towards bottom 131. A spline width (w) and spline thickness (t) that taper towards bottom 131, as an outer perimeter of earplug 100 also tapers towards first end 101, facilities a flange cavity having sufficient collapsible volume for flange 124 to collapse into.

An earplug 100 having geometric features, such as splines 150, dimensioned as described above, provides sufficient thickness to achieve desired sound attenuation and to be maintained in the ear canal of a user while readily compressing and/or collapsing during insertion. In an exemplary embodiment, splines 150 provide an increased surface area of interior flange surface 125, as compared to an interior surface without splines or other geometric features, and promote collapse of flange 124 in desired locations that result in extensional forces or tension in exterior flange surface 125. Extensional forces or tension in exterior flange surface 125 maintains a smooth surface substantially free of creases and/or folds that could otherwise result in reduced acoustic attenuation of earplug 100. Accordingly, splines 150, as described herein reduce insertion forces required to position earplug 100 in the ear canal, reduce equilibrium force to improve comfort in an ear canal, particularly when worn for extended periods of time, and promote desired attenuation by minimizing folds or creases in exterior flange surface 125.

Flange cavity 130 of earplug 100 may be characterized by volume. In an exemplary embodiment, flange cavity has an open volume (V) defined by interior flange surface 126, outer stem surface 114 of stem 110, and cavity plane 13 and a base end plane 15 transverse to longitudinal axis 10 at base end 122 of flange 124. In an exemplary embodiment, (V) is between 500 mm³ and 600 mm³, 525 mm³ and 575 mm³, or about 550 mm³. A volume (V), and flange thicknesses and aspect ratio (R) as described herein, provides an appropriate volume to allow compression and collapse of flange 124 into flange cavity 130 such that a earplug 100 may be comfortably worn while providing a desired level of sound attenuation for a wide range of users having varied ear canal shapes and sizes.

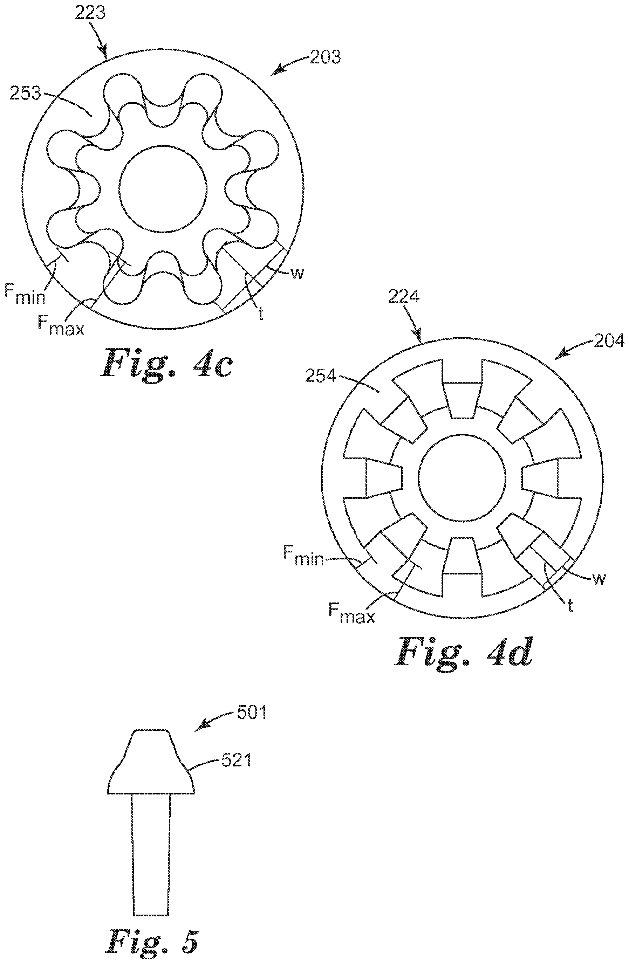

Geometric features such as splines 150 may have any suitable shape that result in stress concentrations at selected locations to affect compression and/or collapse of flange 124. In an exemplary embodiment of Figs. 1 through 3, splines 150 exhibit a substantially triangular cross-section, and are only one example of many suitable shapes for splines as described herein. Other exemplary shapes, include, but are not limited to, the shapes of FIGS. 4a through 4d. FIGS. 4a through 4d show earplugs 201, 202, 203, 204 having flanges 221, 222, 223, and 224 and splines 251, 252, 253, 254 that exhibit square, arcuate, hemispherical or gear tooth cross-sectional shapes, respectively. Flanges 221, 222, 223, and 224 and splines 251, 252, 253, 254 may be characterized by a maximum flange thickness (Fmax), minimum flange thickness (Fmin), spline width (w), and spline thickness (t), as described further herein with respect to splines 150, for example.

An exemplary earplug as described herein may have any suitable shape or profile to provide a desired fit or that may be suitable for a particular application. The specific shape of sound attenuating body 120 of the exemplary embodiment depicted in FIGS. 1 through 3 is only one example of a potentially suitable shape for an earplug as described herein. Examples of one of the myriad of alternative shapes that could be used for earplugs as described herein is depicted in FIG. 5 showing exemplary earplug 500 having sound attenuating body 521.

In some exemplary embodiments, a channel 113 extends through earplug 100 between first end 101 and second end 102. Earplugs as described herein that include channels passing through the earplug may be manufactured such that components of a receiver or of a communication system may be attached to the earplug. Alternatively or in addition, channel 113 may accommodate one or more filters or other passive hearing elements to provide an attenuation curve having a desired shape. For example, filters positioned in channel 113 may cause nonlinear attenuation of high level impulses produced by explosions, gunfire, or the like. Channels provided in one or more embodiments of earplugs as described herein may also provide a recess that a cord may be attached to, such that first and second earplugs may be joined, or that ends of a headband may be attached to in a semi-aural hearing protector.

Earplugs as described herein may be manufactured in any suitable manner. In an exemplary embodiment, earplug 100 includes a core 140 that provides a substrate onto which an outer layer of material may be provided and, in one or more embodiments, may facilitate insertion into an ear canal of a user. Core 140 is made of a first material that exhibits greater rigidity or stiffness than a second material that forms sound attenuating body 120, yet that is soft enough to be comfortable and safe for a user. In an exemplary embodiment, the first material of core 140 is different than the second material used to form sound attenuating body 120 and/or an outer layer 115 of stem 110. In other exemplary embodiments, the first and second materials are similar or the same chemically, but may be formed or provided in a manner that results in different stiffnesses between the first material and the second material, for example due to differing density, cell structure, hardness, etc.

Including a core 140 in the stem 110 that is stiffer than the material of sound attenuating body 120 and/or outer layer 115 of the stem 110 may, in one or more embodiments, provide a stem 110 having sufficient rigidity so that the earplugs described herein may be positioned for use at least partially in the ear of a user by pushing sound attenuating body 120 into the ear canal with an appropriate force. That is, a sufficiently stiff stem 110 may be provided by a core 140 and an outer layer 115 of the material used to form the sound attenuating body 120 so that earplug 100 may be positioned for use at least partially in the ear of a user without the need to first compress or "roll down" sound attenuating body 120. Direct insertion without the need to first compress or "roll down" sound attenuating body 120 may, for example, promote hygiene by limiting contact with sound attenuating body 120 prior to placement in the ear. In one or more embodiments, core 140 may also exhibit an appropriate level of flexibility such that it may slightly deform to follow the contours of the ear canal when positioned for use.

Core 140 may, in one or more embodiments, be made from one or more materials that can suitably bond to, and are otherwise compatible with, the material used to form the sound attenuating body 120 and/or, when present, outer layer 115 of stem 110. In one or more embodiments, core 140 may be made from a blend of polypropylene and styrene-ethylene-butylene-styrene (SEBS), such as TUFPRENE tm available from S&E Specialty Polymers, LLC. of Lunenburg, Massachusetts, or PPC1TF2 tm available from Washington Penn Plastic Co., Inc. of Washington, Pennsylvania. Other potentially suitable materials include SANTOPRENE tm 101-90, available from Exxon Mobile Corporation, and other materials exhibiting appropriate stiffness such that sound attenuating body 120 of earplug 100 may be easily inserted into the ear canal of a user.

A second material used to form sound attenuating body and, in one or more embodiments, an outer layer of a stem of an earplug as described herein, may be soft and pliable foam, rubber, polymer, or other suitable material that may be comfortably positioned in an ear canal of a user. In one or more exemplary embodiments, the second material is an SEBS, such as MONPRENE MP1900 ^{tm} available from Teknor Apex of Pawtucket, Rhode Island, or a blend of high and low molecular weight Kraton SEBS resins resulting in a hardness of 32 Shore A, available from Kraton Polymers LLC, of Houston, Texas, that provides a cellular foam. Other suitable materials include plasticized polyvinyl chloride, ethylene propylene diene monomer (EPDM) rubber, styrene butadiene rubber (SBR), butyl rubber, natural rubbers, other thermoplastics, thermoset polymers, and other suitable materials as known in the art that can be formulated to exhibit an appropriate hardness range.

In one or more embodiments, the materials used to construct a core and sound attenuating body may be selected such that the primary source of bonding between the core and material used for the sound attenuating body (directly or indirectly) is thermal bonding. In one or more embodiments, an additional adhesive is not required to bond the core to the sound attenuating body and, as a result, an adhesive is not present between the core and the sound attenuating body. Although the sound attenuating body of an earplug as described herein may be described as being constructed of a second material, in one or more embodiments a sound attenuating body may be constructed of multiple layers of the same or different materials (which may be, e.g., arranged concentrically). For example, a first layer may be used to provide desired characteristics for contacting an ear canal of a user and a second layer may be used to facilitate a robust bond with the core, while one or more additional layers may be used to provide other desired characteristics.

Materials used in the sound attenuating body of earplugs as described herein may be selected to control the friability of the outer surface of the sound attenuating bodies such that it may not easily be broken or disintegrate during use. The friability of an earplug may be controlled in part by selecting a material having an appropriate molecular weight, with higher molecular weight generally resulting in a less friable earplug. In an exemplary embodiment, sound attenuating body 120 includes an SEBS having a molecular weight between 100,000 Daltons and 200,000 Daltons, as measured by gel permeation chromatography analysis as known in the art, such as according to ASTM D6474 - 99.

The density of outer layer of second material used in the sound attenuating bodies as used in earplugs as described herein can, in one or more embodiments, be controlled during manufacturing to provide a specified density as desired for a particular application. The second material may, in one or more embodiments, exhibit a density that varies by thickness, for example, such that the second material used in the sound attenuating body has an integral outer skin that has a higher density than the second material located closer to the core. Such a skin may be present on one or both of sound attenuating body and the stem (in embodiments in which the stem includes, for example, a layer of the second material used in the sound attenuating body). Alternatively, the second material used to construct the sound attenuating body and/or an outer layer of the stem may have a substantially uniform density.

U.S. Patent Application Ser. No. 13/547,189, titled *Method of Making an Earplug,* addresses a method of making personal protective equipment such as a push-to-fit earplug, U.S. Patent Application Ser. No. 13/547,177, titled *Push-In Earplug,* addresses the structure and configuration of a push-to-fit earplug, and U.S. Patent Application Ser. No. 13/547,294, titled *Foamable Article,* addresses an article for forming a device or component.

In some exemplary embodiments, earplug 100 may be formed from a single material or first and second materials that are similar or the same chemically, but formed or provided in a manner that results in different stiffnesses between the first material and the second material, for example due to differing density, cell structure, hardness, etc. For example, a stem and sound attenuating body having differing properties may be formed by controlling venting in a molding process, and a core 140 may not be included. U.S. Application Ser. No. 61/925,770, *Molded Foam Push-To-Fit Earplug, Method, and* Devices, describes techniques for making an earplug having a sound attenuating body and stiffer stem formed from the same or similar materials.

In various exemplary embodiments, stem 110 and sound attenuating body 120 may be formed separately and subsequently joined together. For example, sound attenuating body 120 may be formed from any suitable may be soft and pliable foam, rubber, polymer, or other suitable material, as described above, and stem 110 may be formed of a more rigid material. Stem 110 and sound attenuating body 120 are then permanently or removably joined, for example by an adhesive, friction, or other engagement.

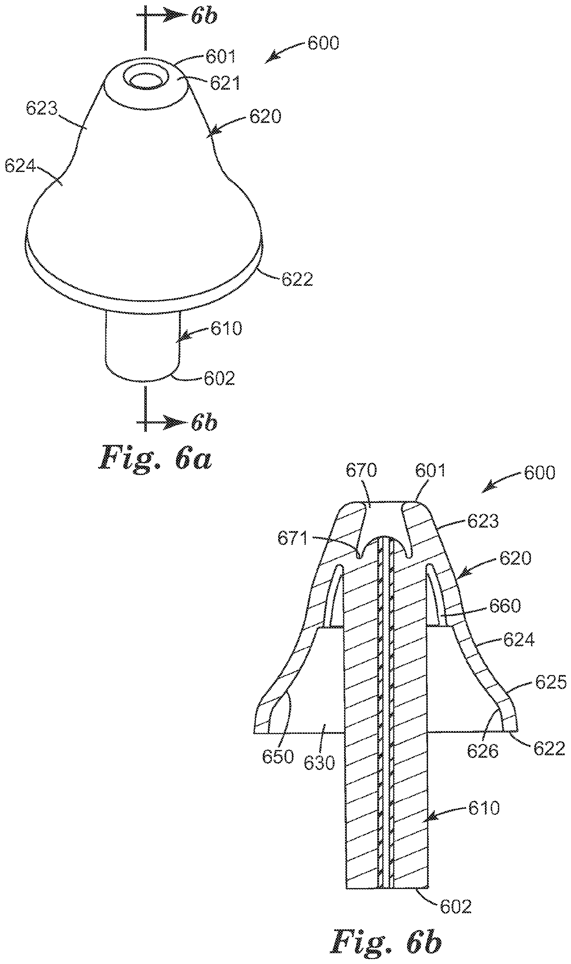

An earplug as described herein may include various other geometric features to enhance comfort or provide improved attenuation. Figures 6a and 6b show an exemplary push-to-fit earplug 600 including a stem 610 and sound attenuating body 620 and having first and second ends 601 and 602. Sound attenuating body 620 includes a leading end 621, a base end 622, a tip region 623 and a flange 624. Tip region 623 is located rearwardly of leading end 621, in front of flange cavity 630, and flange 624 is located between tip region 623 and base end 622. Similar to exemplary earplug 100, flange 622 includes an interior flange surface 626 having a plurality of geometric features, such as splines 650, that may be configured to promote buckling and/or folding of interior flange surface 626, and/or stretching and tensioning of exterior flange surface 625, as described herein.

Exemplary earplug 600 includes a tip cavity 670 that extends from first end 601 of earplug 600 towards a bottom 671 located nearer second end 602 of earplug 600. Tip cavity 670 includes an opening at first end 601 of earplug 600. Tip cavity 670 may, in one or more exemplary embodiments, provide a volume into which the surrounding material of sound attenuating body 620, and particularly tip region 621, can collapse as earplug 600 is advanced into an earcanal and/or is positioned therein. U.S. Application Ser. No. 13/768,214, *Earplug with Tip Cavity and Methods of Manufacturing the Same* address earplugs having a tip cavity.

In an exemplary embodiment, earplug 600 further includes sound attenuating portion 620 includes an array of cavities 660 positioned within tip region 623 and spaced around the longitudinal axis 10. Cavities 660 provide a collapsible volume that at least a portion of sound attenuating portion 620 may collapse into as earplug 100 is advanced into an ear canal of a user. In various exemplary embodiments, array of cavities may include between 4 and 16, 6 and 12, or 8 cavities. U.S. Application Ser. No. 14/282,266, *Push-To-Fit Earplug Having an Array of Cavities,* addresses earplugs having a plurality of inwardly projecting geometric features. .

The present invention has now been described with reference to several embodiments thereof. The foregoing detailed description and examples have been given for clarity of understanding only. No unnecessary limitations are to be understood therefrom. It will be apparent to those skilled in the art that many changes can be made in the embodiments described without departing from the scope of the invention. Thus, the scope of the present invention should not be limited to the exact details and structures described herein, but rather by the structures described by the language of the claims, and the equivalents of those structures. Any patent literature cited herein is hereby incorporated herein by reference in its entirety to the extent that it does not conflict with the description presented herein.

Any feature or characteristic described with respect to any of the above embodiments can be incorporated individually or in combination with any other feature or characteristic, and are presented in the above order and combinations for clarity only. That is, the present disclosure contemplates all possible combinations and arrangements of various features of each of the exemplary embodiments and components describe herein, and each component may be combined or used in conjunction with any other component as may be desired for a particular application.

## Claims

1. An earplug (100; 600), comprising:
a stem (110; 610) extending from a first end (101; 601) to a second end (102; 602) along a longitudinal axis;
a sound attenuating body (120; 620) attached to the stem (110; 610), the sound attenuating body (120; 620) comprising a leading end (121; 621), a base end (122; 622), the longitudinal axis extending between the leading end (121; 621) and the base end (122; 622), and a flange (124; 624) extending at least partially over the stem (110; 610) and comprising an exterior flange surface (125; 625) and an interior flange surface (126; 626) having a plurality of one or both of protrusions or recesses (150; 650); and
a flange cavity (130; 630) comprising a continuous volume around a perimeter of the stem (110; 610) between the interior flange surface (126; 626) and the stem (110; 610), wherein the plurality of one or both of protrusions or recesses (150; 650) extend from the base end (122; 622) of the sound attenuating body (120; 620) to a bottom (131) of the flange cavity (130; 630);
wherein a distance between the interior and exterior flange surfaces (125, 126; 625, 626) varies around a perimeter of the flange (124; 624) at a plane intersecting the flange (124; 624) transverse to the longitudinal axis.

2. The earplug (100; 600) of claim 1, wherein the flange (124; 624) has a minimum flange thickness (Fmin) and a maximum flange thickness (Fmax) between the exterior flange surface (125; 625) and the interior flange surface (126; 626) at the plane intersecting the flange (124; 624) transverse to the longitudinal axis, and wherein 1.5 mm < (Fmax) < 5 mm and 0.5 mm < (Fmin) < 1.5 mm.

3. The earplug (100; 600) of claim 2, wherein 2.0 mm < (Fmax) < 4.5 mm.

4. The earplug (100; 600) of claim 2, wherein 2.0 mm < (Fmax) < 4.5 mm proximate a base end of the flange (124; 624).

5. The earplug (100; 600) of claim 1, wherein the flange (124; 624) has a minimum flange thickness (Fmin) and a maximum flange thickness (Fmax) between the exterior flange surface (125; 625) and the interior flange surface (126; 626) at the plane intersecting the flange (124; 624) transverse to the longitudinal axis, and wherein (Fmax) and (Fmin) vary between a bottom (131) of the flange cavity (130; 630) and a base end of the flange (124; 624).

6. The earplug (100; 600) of claim 1, wherein the flange (124; 624) has a minimum flange thickness (Fmin) and a maximum flange thickness (Fmax) between the exterior flange surface (125; 625) and the interior flange surface (126; 626) at the plane intersecting the flange (124; 624) transverse to the longitudinal axis, and wherein the flange (124; 624) comprises protrusions that exhibit a width (w) measured between two adjacent locations of minimum flange thickness (Fmin) at the plane intersecting the flange (124; 624) transverse to a longitudinal axis of the stem (110; 610), and 2 mm < (w) < 8 mm.

7. The earplug (100; 600) of claim 6, wherein 2 mm < (w) < 8 mm proximate a base end of the flange (124; 624).

8. The earplug (100; 600) of claim 1, wherein the flange (124; 624) has a minimum flange thickness (Fmin) and a maximum flange thickness (Fmax) between the exterior flange surface (125; 625) and the interior flange surface (126; 626) at the plane intersecting the flange (124; 624) transverse to the longitudinal axis, and wherein the flange (124; 624) comprises protrusions that exhibit a width (w) measured between two adjacent locations of minimum flange thickness (Fmin) at the plane intersecting the flange (124; 624) transverse to a longitudinal axis of the stem (110; 610), and wherein the flange (124; 624) exhibits a flange aspect ratio (w/Fmax), and .75 < (w/Fmax) < 3.

9. The earplug (100; 600) of claim 1, wherein in a neutral configuration the interior flange surface (126; 626) does not contact the stem (110; 610), and in a compressed configuration the interior flange surface (126; 626) at least partially contacts the stem (110; 610).

10. The earplug (100; 600) of claim 1, wherein the flange (124; 624) is configured to collapse into the flange cavity (130; 630) such that the interior flange surface (126; 626) at least partially contacts the stem (110; 610).

11. The earplug (100; 600) of claim 1, wherein the interior flange surface (126; 626) comprises a shape selected from the group consisting of a triangular shape, arcuate shape, gear tooth shape, and quadrilateral shape.

12. The earplug (100; 600) of claim 1, wherein the stem (110; 610) comprises a core (140) made of a first material and an outer layer (115) made of a second material.

13. The earplug (100; 600) of claim 12, wherein the core comprises a material selected from the group consisting of polypropylene and styrene-ethylene-butylene-styrene (SEBS).

14. The earplug (100; 600) of claim 1, wherein the sound attenuating body (120; 620) comprises a foam.

15. The earplug (100; 600) of claim 1, wherein the stem (110; 610) comprises a core (140) made of a first material and an outer layer (115) made of a second material, wherein the flange (124; 624) has a minimum flange thickness (Fmin) and a maximum flange thickness (Fmax) between the exterior flange surface (125; 625) and the interior flange surface (126; 626) at the plane passing through the flange (124; 624) transverse to the longitudinal axis, and wherein the flange (124; 624) comprises protrusions that exhibit a width (w) measured between two adjacent locations of minimum flange thickness (Fmin) at the plane intersecting the flange (124; 624) transverse to a longitudinal axis of the stem (110; 610), and wherein 1.5 mm < (Fmax) < 5 mm, 0.5 mm < (Fmin) < 1.5 mm, and 2 mm < (w) < 8 mm.

## Patentansprüche

1. Ein Ohrstöpsel (100; 600), umfassend:
einen Schaft (110; 610), der sich von einem ersten Ende (101; 601) zu einem zweiten Ende (102; 602) entlang einer Längsachse erstreckt;
einen Schalldämpfungskörper (120; 620), der an dem Schaft (110; 610) befestigt ist, wobei der Schalldämpfungskörper (120; 620) ein vorderes Ende (121; 621), ein Basisende (122; 622), eine sich zwischen dem vorderen Ende (121; 621) und dem Basisende (122; 622) erstreckende Längsachse und einen sich zumindest teilweise über den Schaft (110; 610) erstreckenden Flansch (124; 624) umfasst und eine äußere Flanschoberfläche (125; 625) und eine innere Flanschoberfläche (126; 626) mit einer Vielzahl von einem oder beiden von Vorsprüngen oder Vertiefungen (150; 650) umfasst; und
einen Flanschhohlraum (130; 630), der ein kontinuierliches Volumen um einen Umfang des Schafts (110; 610) herum zwischen der inneren Flanschoberfläche (126; 626) und dem Schaft (110; 610) umfasst, wobei sich die Vielzahl von einem oder beiden der Vorsprünge oder Vertiefungen (150; 650) von dem Basisende (122; 622) des Schalldämpfungskörpers (120; 620) bis zu einem Boden (131) des Flanschhohlraums (130; 630) erstreckt;
wobei ein Abstand zwischen der inneren und der äußeren Flanschoberfläche (125, 126; 625, 626) um einen Umfang des Flansches (124; 624) in einer Ebene variiert, die den Flansch (124; 624) quer zur Längsachse schneidet.

2. Der Ohrstöpsel (100; 600) nach Anspruch 1, wobei der Flansch (124; 624) eine minimale Flanschdicke (Fmin) und eine maximale Flanschdicke (Fmax) zwischen der äußeren Flanschoberfläche (125; 625) und der inneren Flanschoberfläche (126; 626) in der Ebene aufweist, die den Flansch (124; 624) quer zur Längsachse schneidet, und wobei 1,5 mm < (Fmax) < 5 mm und 0,5 mm < (Fmin) < 1,5 mm ist.

3. Der Ohrstöpsel (100; 600) nach Anspruch 2, wobei 2,0 mm < (Fmax) < 4,5 mm ist.

4. Der Ohrstöpsel (100; 600) nach Anspruch 2, wobei nahe einem Basisende des Flansches (124; 624) 2,0 mm < (Fmax) < 4,5 mm ist.

5. Der Ohrstöpsel (100; 600) nach Anspruch 1, wobei der Flansch (124; 624) eine minimale Flanschdicke (Fmin) und eine maximale Flanschdicke (Fmax) zwischen der äußeren Flanschoberfläche (125; 625) und der inneren Flanschoberfläche (126; 626) in der Ebene, die den Flansch (124; 624) quer zur Längsachse schneidet, aufweist und wobei (Fmax) und (Fmin) zwischen einem Boden (131) des Flanschhohlraums (130; 630) und einem Basisende des Flansches (124; 624) variieren.

6. Der Ohrstöpsel (100; 600) nach Anspruch 1, wobei der Flansch (124; 624) eine minimale Flanschdicke (Fmin) und eine maximale Flanschdicke (Fmax) zwischen der äußeren Flanschoberfläche (125; 625) und der inneren Flanschoberfläche (126; 626) in der Ebene aufweist, die den Flansch (124; 624) quer zur Längsachse schneidet, und wobei der Flansch (124; 624) Vorsprünge umfasst, die eine Breite (w) aufweisen, die zwischen zwei benachbarten Stellen minimaler Flanschdicke (Fmin) in der Ebene gemessen wird, die den Flansch (124; 624) quer zu einer Längsachse des Schafts (110; 610) schneidet, und 2 mm < (w) < 8 mm ist.

7. Der Ohrstöpsel (100; 600) nach Anspruch 6, wobei nahe eines Basisendes des Flansches (124; 624) 2 mm < (w) < 8 mm ist.

8. Der Ohrstöpsel (100; 600) nach Anspruch 1, wobei der Flansch (124; 624) eine minimale Flanschdicke (Fmin) und eine maximale Flanschdicke (Fmax) zwischen der äußeren Flanschoberfläche (125; 625) und der inneren Flanschoberfläche (126; 626) in der Ebene aufweist, die den Flansch (124; 624) quer zur Längsachse schneidet, und wobei der Flansch (124; 624) Vorsprünge umfasst, die eine Breite (w) aufweisen, die zwischen zwei benachbarten Stellen minimaler Flanschdicke (Fmin) in der Ebene gemessen wird, die den Flansch (124; 624) quer zu einer Längsachse des Schafts (110; 610) schneidet, und wobei der Flansch (124; 624) ein Flansch-Seitenverhältnis (w/Fmax) aufweist und 0,75 < (w/Fmax) < 3 ist.

9. Der Ohrstöpsel (100; 600) nach Anspruch 1, wobei in einer neutralen Konfiguration die innere Flanschoberfläche (126; 626) den Schaft (110; 610) nicht berührt und in einer komprimierten Konfiguration die innere Flanschoberfläche (126; 626) den Schaft (110; 610) zumindest teilweise berührt.

10. Der Ohrstöpsel (100; 600) nach Anspruch 1, wobei der Flansch (124; 624) dafür konfiguriert ist, in den Flanschhohlraum (130; 630) zusammenzufallen, so dass die innere Flanschoberfläche (126; 626) den Schaft (110; 610) zumindest teilweise berührt.

11. Der Ohrstöpsel (100; 600) nach Anspruch 1, wobei die innere Flanschoberfläche (126; 626) eine Form ausgewählt aus der Gruppe bestehend aus einer dreieckigen Form, einer bogenförmigen Form, einer Zahnradzahnform und einer vierseitigen Form umfasst.

12. Der Ohrstöpsel (100; 600) nach Anspruch 1, wobei der Schaft (110; 610) einen Kern (140) aus einem ersten Material und eine Außenschicht (115) aus einem zweiten Material umfasst.

13. Der Ohrstöpsel (100; 600) nach Anspruch 12, wobei der Kern ein Material ausgewählt aus der Gruppe bestehend aus Polypropylen und Styrol-Ethylen-Butylen-Styrol (SEBS) umfasst.

14. Der Ohrstöpsel (100; 600) nach Anspruch 1, wobei der Schalldämpfungskörper (120; 620) einen Schaumstoff umfasst.

15. Der Ohrstöpsel (100; 600) nach Anspruch 1, wobei der Schaft (110; 610) einen Kern (140) aus einem ersten Material und eine Außenschicht (115) aus einem zweiten Material umfasst, wobei der Flansch (124; 624) eine minimale Flanschdicke (Fmin) und eine maximale Flanschdicke (Fmax) zwischen der äußeren Flanschoberfläche (125; 625) und der inneren Flanschoberfläche (126; 626) in der Ebene, die durch den Flansch (124; 624) quer zur Längsachse verläuft, aufweist und wobei der Flansch (124; 624) Vorsprünge umfasst, die eine Breite (w) aufweisen, die zwischen zwei benachbarten Stellen minimaler Flanschdicke (Fmin) in der Ebene, die den Flansch (124; 624) quer zu einer Längsachse des Schafts (110; 610) schneidet, gemessen wird, und wobei 1,5 mm < (Fmax) < 5 mm, 0,5 mm < (Fmin) < 1,5 mm und 2 mm < (w) < 8 mm ist.

## Revendications

1. Bouchon d'oreille (100 ; 600), comprenant :
une tige (110 ; 610) s'étendant depuis une première extrémité (101 ; 601) jusqu'à une deuxième extrémité (102 ; 602) le long d'un axe longitudinal ;
un corps atténuateur de bruit (120 ; 620) fixé à la tige (110 ; 610), le corps atténuateur de bruit (120 ; 620) comprenant une extrémité avant (121 ; 621), une extrémité de base (122 ; 622), l'axe longitudinal s'étendant entre l'extrémité avant (121 ; 621) et l'extrémité de base (122 ; 622), et un rebord (124 ; 624) s'étendant au moins partiellement par-dessus la tige (110 ; 610) et comprenant une surface extérieure de rebord (125 ; 625) et une surface intérieure de rebord (126 ; 626) ayant une pluralité de l'un et/ou l'autre parmi des parties saillantes ou des renfoncements (150 ; 650) ; et
une cavité de rebord (130 ; 630) comprenant un volume continu autour d'un périmètre de la tige (110 ; 610) entre la surface intérieure de rebord (126 ; 626) et la tige (110 ; 610), dans lequel la pluralité de l'un et/ou l'autre parmi les parties saillantes ou les renfoncements (150 ; 650) s'étendent depuis l'extrémité de base (122 ; 622) du corps atténuateur de bruit (120 ; 620) jusqu'à un fond (131) de la cavité de rebord (130 ; 630) ;
dans lequel une distance entre les surfaces intérieure et extérieure de rebord (125, 126 ; 625, 626) varie autour d'un périmètre du rebord (124 ; 624) au niveau d'un plan croisant le rebord (124 ; 624) transversalement à l'axe longitudinal.

2. Bouchon d'oreille (100 ; 600) selon la revendication 1, dans lequel le rebord (124 ; 624) a une épaisseur minimale de rebord (Fmin) et une épaisseur maximale de rebord (Fmax) entre la surface extérieure de rebord (125 ; 625) et la surface intérieure de rebord (126 ; 626) au niveau du plan croisant le rebord (124 ; 624) transversalement à l'axe longitudinal, et dans lequel 1,5 mm < (Fmax) < 5 mm et 0,5 mm < (Fmin) < 1,5 mm.

3. Bouchon d'oreille (100 ; 600) selon la revendication 2, dans lequel 2,0 mm < (Fmax) < 4,5 mm.

4. Bouchon d'oreille (100 ; 600) selon la revendication 2, dans lequel 2,0 mm < (Fmax) < 4,5 mm à proximité d'une extrémité de base du rebord (124 ; 624).

5. Bouchon d'oreille (100 ; 600) selon la revendication 1, dans lequel le rebord (124 ; 624) a une épaisseur minimale de rebord (Fmin) et une épaisseur maximale de rebord (Fmax) entre la surface extérieure de rebord (125 ; 625) et la surface intérieure de rebord (126 ; 626) au niveau du plan croisant le rebord (124 ; 624) transversalement à l'axe longitudinal, et dans lequel (Fmax) et (Fmin) varient entre un fond (131) de la cavité de rebord (130 ; 630) et une extrémité de base du rebord (124 ; 624).

6. Bouchon d'oreille (100 ; 600) selon la revendication 1, dans lequel le rebord (124 ; 624) a une épaisseur minimale de rebord (Fmin) et une épaisseur maximale de rebord (Fmax) entre la surface extérieure de rebord (125 ; 625) et la surface intérieure de rebord (126 ; 626) au niveau du plan croisant le rebord (124 ; 624) transversalement à l'axe longitudinal, et dans lequel le rebord (124 ; 624) comprend des parties saillantes qui présentent une largeur (w) mesurée entre deux emplacements adjacents d'épaisseur minimale de rebord (Fmin) au niveau du plan croisant le rebord (124 ; 624) transversalement à un axe longitudinal de la tige (110 ; 610), et 2 mm < (w) < 8 mm.

7. Bouchon d'oreille (100 ; 600) selon la revendication 6, dans lequel 2 mm < (w) < 8 mm à proximité d'une extrémité de base du rebord (124 ; 624).

8. Bouchon d'oreille (100 ; 600) selon la revendication 1, dans lequel le rebord (124 ; 624) a une épaisseur minimale de rebord (Fmin) et une épaisseur maximale de rebord (Fmax) entre la surface extérieure de rebord (125 ; 625) et la surface intérieure de rebord (126 ; 626) au niveau du plan croisant le rebord (124 ; 624) transversalement à l'axe longitudinal, et dans lequel le rebord (124 ; 624) comprend des parties saillantes qui présentent une largeur (w) mesurée entre deux emplacements adjacents d'épaisseur minimale de rebord (Fmin) au niveau du plan croisant le rebord (124 ; 624) transversalement à un axe longitudinal de la tige (110 ; 610), et dans lequel le rebord (124 ; 624) présente un rapport d'aspect de rebord (w/Fmax), et 0,75 < (w/Fmax) <3.

9. Bouchon d'oreille (100 ; 600) selon la revendication 1, dans lequel dans une configuration neutre la surface intérieure de rebord (126 ; 626) ne vient pas en contact avec la tige (110 ; 610), et dans une configuration compressée la surface intérieure de rebord (126 ; 626) vient au moins partiellement en contact avec la tige (110 ; 610).

10. Bouchon d'oreille (100 ; 600) selon la revendication 1, dans lequel le rebord (124 ; 624) est configuré pour s'écraser dans la cavité de rebord (130 ; 630) de telle sorte que la surface intérieure de rebord (126 ; 626) vient au moins partiellement en contact avec la tige (110 ; 610).

11. Bouchon d'oreille (100 ; 600) selon la revendication 1, dans lequel la surface intérieure de rebord (126 ; 626) comprend une forme choisie dans le groupe constitué d'une forme triangulaire, d'une forme arquée, d'une forme de dent d'engrenage, et d'une forme quadrilatérale.

12. Bouchon d'oreille (100 ; 600) selon la revendication 1, dans lequel la tige (110 ; 610) comprend un noyau (140) constitué d'un premier matériau et une couche externe (115) constituée d'un deuxième matériau.

13. Bouchon d'oreille (100 ; 600) selon la revendication 12, dans lequel le noyau comprend un matériau choisi dans le groupe constitué de polypropylène et de styrène-éthylène-butylène-styrène (SEBS).

14. Bouchon d'oreille (100 ; 600) selon la revendication 1, dans lequel le corps atténuateur de bruit (120 ; 620) comprend une mousse.

15. Bouchon d'oreille (100 ; 600) selon la revendication 1, dans lequel la tige (110 ; 610) comprend un noyau (140) constitué d'un premier matériau et une couche externe (115) constituée d'un deuxième matériau, dans lequel le rebord (124 ; 624) a une épaisseur minimale de rebord (Fmin) et une épaisseur maximale de rebord (Fmax) entre la surface extérieure de rebord (125 ; 625) et la surface intérieure de rebord (126 ; 626) au niveau du plan traversant le rebord (124 ; 624) transversalement à l'axe longitudinal, et dans lequel le rebord (124 ; 624) comprend des parties saillantes qui présentent une largeur (w) mesurée entre deux emplacements adjacents d'épaisseur minimale de rebord (Fmin) au niveau du plan croisant le rebord (124 ; 624) transversalement à un axe longitudinal de la tige (110 ; 610), et dans lequel 1,5 mm < (Fmax) < 5 mm, 0,5 mm < (Fmin) < 1,5 mm, et 2 mm < (w) < 8 mm.
